Europäisches Patentamt

European Patent Office

Office européen des brevets

(11) Veröffentlichungsnummer : **0 069 307**
**B1**

# EUROPÄISCHE PATENTSCHRIFT

(45) Veröffentlichungstag der Patentschrift :
05.03.86

(21) Anmeldenummer : 82105663.7

(22) Anmeldetag : 25.06.82

(51) Int. Cl.⁴ : **A 61 K   9/50**

(54) **Verfahren zur Herstellung von Liposomenlösungen.**

(30) Priorität : 02.07.81 CH 4376/81

(43) Veröffentlichungstag der Anmeldung :
12.01.83 Patentblatt 83/02

(45) Bekanntmachung des Hinweises auf die Patenterteilung : 05.03.86 Patentblatt 86/10

(84) Benannte Vertragsstaaten :
AT BE CH DE FR GB IT LI NL SE

(56) Entgegenhaltungen :
EP-A- 0 043 327
GB-A- 1 490 955
CHEMICAL ABSTRACTS, Band 85, Nr. 4, 26. Juli
1976, Seite 258, Nr. 25402a, COLUMBUS OHIO (US).
JOURNAL OF THE CHEMICAL SOCIETY, Faraday
Transactions I; Teil 2, Februar 1982; Seiten 323-339;
J. MININGS et al :"Phospholipid monolayers at nonpolar oil/water Interfaces"

(73) Patentinhaber : **F. HOFFMANN-LA ROCHE & CO.**
**Aktiengesellschaft**

**CH-4002 Basel (CH)**

(72) Erfinder : **Heyne, Christian, Dr.**
**Am Schlossberg 18**
**D-7801 Au/Brsg (DE)**

(74) Vertreter : **Lederer, Franz, Dr. et al**
**Patentanwälte Dr. Franz Lederer Dipl.-Ing. Reiner F.**
**Meyer-Roxlau Lucile-Grahn-Strasse 22**
**D-8000 München 80 (DE)**

## Beschreibung

Die vorliegende Erfindung betrifft ein Verfahren zur Herstellung von Liposomenlösungen, die einen pharmakologischen Wirkstoff bzw. Stabilisatoren und Antioxidantien enthalten können.

Das erfindungsgemässe Verfahren ist dadurch gekennzeichnet, dass man ein wässriges System, das einen Liposomenbildner und ein inertes flüchtiges organisches Lösungsmittel oder ein Gemisch solcher Lösungsmittel enthält, mit Ultraschall behandelt, wobei man gegebenenfalls vor der Beschallung einen pharmakologischen Wirkstoff bzw. Stabilisatoren und Antioxidantien zusetzt.

Als Liposomenbildner kommen Lipide, insbesondere Phospholipide, wie Lecithine in Betracht. Die Lecithine können pflanzlicher, tierischer oder synthetischer Art sein. Beispiele von Lecithinen sind Sojalecithin, Eilecithin oder L-β-Oleoyl-γ-palmitoyl-α-lecithin. Als Liposomenbildner können auch Gemische von Phospholipiden und Adjuvantien, wie Triglyceride, Cholesterine, Cholesterinester, Antioxidantien (z. B. α-Tocopherol) und Zucker, verwendet werden.

Beispiele inerter, flüchtiger organischer Lösungsmittel, die auch als Gemische eingesetzt werden können, sind niedere aliphatische Alkohole, wie Aethanol, Propanol und Isopropanol; sowie Aether, insbesondere Diäthyläther.

Ultraschall wurde bisher bei der Herstellung von Liposomen zur mechanischen Zerkleinerung von bereits vorgebildeten multilamellaren Vesikeln angewandt. Mit diesen bekannten Verfahren konnten jedoch nur Liposomenlösungen in verhältnismässig niedriger Konzentration hergestellt werden. Weitere Nachteile bestanden in der starken Erwärmung der Präparate, hohem Energiebedarf, Materialabrieb am Ultraschallgerät und schlechter Durchführbarkeit im grosstechnischen Massstab.

Aus der GB-A-1 490 955 ist ein Verfahren zur Herstellung von Liposomen bekannt, bei dem von einem System ausgegangen wird, in dem eine organische Phase überwiegt. Bei diesem bekannten Verfahren muss nach der in erster Verfahrensstufe durchgeführten Ultraschallbehandlung in einer zweiten Verfahrensstufe zentrifugiert werden. In der genannten GB-PS wird weiterhin ein Verfahren zur Herstellung von Liposomenlösungen erwähnt, bei dem der Liposomenbildner zunächst in einem flüchtigen Lösungsmittel gelöst wird, worauf dieses verdampft, der von den Liposomen einzuschliessende Stoff zugesetzt und das so erhaltene System beschallt wird.

Das erfindungsgemässe Verfahren vermeidet Nachteile bekannter Verfahren, indem durch Schwankungen des lokalen Drucks im System, wie sie beispielsweise durch Ultraschall bewirkt werden, das Löslichkeitsgleichgewicht einer inerten Hilfskomponente und das damit gekoppelte Phasengleichgewicht, z. B. $Gas_{(gasförmig)} \rightleftharpoons Gas_{(solvatisiert)}$ zur Bildung der Liposomen ausgenutzt werden. Die lokalen Druckschwankungen führen zu einer periodisch auftretenden Phasentrennung, z. B. durch Ausgasen und Wiederverdichten bzw. Lösen der Hilfskomponente (d. h. des flüchtigen organischen Lösungsmittels), die wiederum die Moleküle des Liposomenbildners zur Ausrichtung um die entstehenden und kollabierenden, submikroskopischen Phasenkompartimente (z. B. Gasbläschen) der Hilfskomponente veranlasst.

In einer bevorzugten Ausführungsform wird das erfindungsgemässe Verfahren mit Lösungen durchgeführt, in denen der Liposomenbildner in einer Konzentration von etwa 20-50 Gew.-% vorliegt. Die Hilfskomponente kann in Mengen bis zur Sättigungskonzentration der Phospholipide anwesend sein. In einer weiter bevorzugten Ausführungsform dient die Hilfskomponente als Lösungsmittel für den Liposomenbildner. Als Ultraschallquelle können die konventionellen Ultraschallgeräte, z. B. Geräte für Ultraschallreinigungsbäder, verwendet werden. Mit Ausgangskonzentrationen von 20-50 % Lecithin erhält man nach kurzen Beschallungszeiten ein schaumiges Gel, das sich in Wasser aufnehmen lässt und dabei eine Liposomenlösung liefert, in der Liposomen von ca. 200 nm Durchmesser und signifikanter Grössenverteilung (Dispersität 2-3, gemessen im KONTRON-NANOSIZER) vorliegen. Das nach kurzer Beschallung konzentrierter Lösungen wie oben beschrieben erhaltene Liposomen-Gel geht nach weiterer Beschallung (ca. 15-30 Minuten) in eine klar-opake Liposomenlösung über, in der die Liposomen je nach Beschallungszeit einen Durchmesser von 40-130 nm und eine ausgezeichnete Monodispersität aufweisen.

Auf diese Weise lassen sich viskose Liposomenlösungen z. B. mit 20-25 Gew.-% Liposomen herstellen, die z. B. ein Filter von 0,22 μ Porenweite passieren. Die überschüssige Hilfskomponente kann z. B. durch Anlegen eines Vakuums entfernt werden.

Das primär erhaltene Liposomengel kann auch als solches Verwendung finden, z. B. eignet es sich zur Verwendung als Salbengrundlage.

Die Liposomenlösungen können z. B. zur Herstellung von Präparaten, insbesondere pharmazeutischen Präparaten Verwendung finden, die einen in Wasser nicht oder schlecht löslichen Wirkstoff enthalten. Beispiele solcher Wirkstoffe sind lipophile pharmakologische Wirkstoffe, wie Benzodiazepine, z. B. Diazepam, Chlordiazepoxid, Flunitrazepam, Medazepam, Clonazepam und Bromazepam; oder fettlösliche Vitamine wie die Vitamine A, D, E und K. Die erfindungsgemässen Liposomenlösungen können auch wasserlösliche Wirkstoffe, z. B. Vitamine der B-Gruppe, wie Vitamin $B_1$ und $B_6$, enthalten; diese Wirkstoffe werden der Lösung des Liposomenbildners (im Falle lipophiler Wirkstoffe) bzw. als wässrige Lösung (im Falle hydrophiler Wirkstoffe) vor der Beschallung zugesetzt.

Die nachfolgenden Beispiele erläutern die Erfindung.

Beispiel 1

400 g Phospholipon 100 (PL 100) werden auf dem Wasserdampfbad in einem genügenden Volumen Aethanol in Lösung gebracht und anschliessend auf 600 g Konzentrat eingeengt. Dieses wird in ein einfaches Laborultraschallgerät (300 Watt, z. B. Compact TUC 300) unter Beschallung eingegeben und mit 2 l stickstoffgesättigtem $H_2O$-bidest. untergerührt. Zur Vermeidung von Oxydationen im Reaktionsgemisch wird ein langsamer Strom von hochgereinigtem Stickstoff über die Lösung geleitet. Schon nach wenigen Minuten entsteht auf diese Weise ein blasiger Gel. Eine Probe wird in überschüssigem Volumen von $H_2O$ dispergiert und ergibt bereits jetzt Liposomen von ausgezeichneter Monodispersität und einem durchschnittlichen Vesikeldurchmesser von 0,20 μm (gemessen im NANOSIZER der Firma KONTRON), Nach einer weiteren Beschallungszeit des Geles von ca. 30 Minuten beginnt die Verflüssigung zu einem hochviskosen, opaleszierenden Präparat, das unter starkem Rühren nach ca. Weiteren 30 Minuten Beschallungszeit in den dünnflüssigen Zustand übergeht. Die von den Sonoren verursachte Eigenkonvektion der Lösung genügt um für die abschliessenden 15 Minuten Beschallungszeit, bis zur Fertigstellung der Liposomenlösung, eine ausreichende Durchmengung zu sichern. Während des gesamten Darstellungsprozesses sollte eine Temperatur von 40 °C nicht überschritten und auf Einhaltung der Inertatmosphäre geachtet werden. Nach Erreichen eines durchschnittlichen Vesikeldurchmessers von ca. 100-120 nm wird die hochprozentige Lösung abgegossen und sukzessive durch ein Membranfilter von 1,2 μm, 0,45 μm und 0,22 μm Porendurchmesser unter Stickstoff hindurch gepresst (ca. 5 at).

Die so erhaltene Liposomenlösung kann sowohl durch Abkühlung unter 0 °C wie auch durch Hitzesterilisation in einen klaren Gel überführt werden. Ist dieser Effekt nicht erwünscht kann durch Zugabe eines Elektrolyten (z. B. physiologisches Puffersystem) bzw. eines Zuckers wie u. a. sorbitol die Gelumwandlung unterdrückt werden.

Beispiel 2

Es wird ein Ansatz nach Beispiel 1 zubereitet, jedoch die Beschallung nach Erreichen des Gelzustandes (ca. 15 Minuten Beschallungszeit) abgebrochen. Der so erhaltene Gel wird mehrere Tage unter Inertgas aufbewahrt. Hiernach wurde unter den gleichen Versuchsbedingungen nach Zugabe von 200 ml stickstoffgesättigtem $H_2O$ weiter beschallt. Schon nach 15-30 Minuten erhält man eine fertige Liposomenlösung von guter Dispersität und einem mittleren Vesikeldurchmesser von 120-130 nm. Die Lösung kann wie im Beispiel 1 filtriert werden.

Beispiel 3

400 g PL 100 werden in ca. 200 ml Diäthyläther gelöst und bis zum Sättigungspunkt eingeengt. Das so erhaltene Konzentrat (ca. 500-600 ml) wird wie bereits beschrieben in das Ultraschallgerät gegeben, mit 2 l stickstoffgesättigtem $H_2O$ überschichtet und unter Inertgas ca. 30 Minuten beschallt. Während der Beschallung werden die auf der Oberfläche flottierenden Teile mittels eines Handmixers zerkleinert und untergerührt. Es entsteht ein hochviskoser, schleimiger Gel, der nach weiterer Beschallung von ca. 15 Minuten eine bernsteinfarbene, gelartige Masse ergibt, welche sich bei weiterer Beschallung unter Blasenentwicklung zu verflüssigen beginnt. Die so erhaltene Liposomenlösung kann auch nach Austreibung des Aethers mittels eingeleitetem Stickstoff nur schwer in $H_2O$ aufgenommen werden (Agglomerisation). Bei Zugabe von einer Mischung aus Aceton und $H_2O$ im Verhältnis 1 : 1 erhält man hingegen eine milchig-opake Lösung, die sich nach Austreibung der organ. Lösungsmittel im Rotationsverdampfer leicht nach Beispiel 1 filtrieren lässt. Statt der Zugabe des Aceton-Wasser-Gemisches zu obigem agglomerisierendem Präparat lässt sich die Aufnahme in $H_2O$ auch durch Zugabe von ca. 100 ml Aethanol erreichen wobei die Lösungsmittel wie beschrieben entfernt werden. Der mittlere Vesikeldurchmesser beträgt 120-130 nm.

Beispiel 4

400 g PL 100 werden in Alkohol gelöst und bis zum Sättigungspunkt eingedampft. Es werden 100 g Natriumbicarbonat und 400 g Traubenzucker eingerührt (statt Traubenzucker lässt sich auch Sorbitol verwenden). Die so erhaltene zähe Masse wird nach bereits beschriebener Weise in das Ultraschallbad gegeben und mit 2 l stickstoffgesättigtem Wasser überschichtet. Unter vorsichtigem Rühren und gleichzeitiger Beschallung werden 100 g Phosphorsäure (80 %) allmählich hinzugetropft. Bei Nachlassen der Gasentwicklung und Schäumung wird nur noch gelegentlich gerührt und nach ca. 60-90 Minuten die fertige Liposomenlösung nach beschriebenem Verfahren gefiltert. Das Präparat ist deutlich getrübt und zeigt eine mittlere Vesikelgrösse von 130 nm und guter Monodispersität. Ein Teil dieser Lösung wurde mit etwa 100 ml Aethanol versetzt und weitere 30 Minuten beschallt. Hierbei nahmen Viskosität und Transparenz deutlich zu, der mittlere Vesikeldurchmesser verringerte sich deutlich auf 110-120 nm.

Beispiel 5

400 g PL 100 werden in 800 ml Aether gelöst und hiernach mit 800 ml Aceton aufgefüllt. Die Lösung wird nach bereits beschriebener Weise in das Ultraschallbad gegeben. Es werden unter Beschallung allmählich 1 l stickstoffgesättigtes Wasser so zugetropft, dass keine Fällung entsteht. Die derart erhaltene honigfarbene, klare leicht viskose Lösung wird ca. 60 Minuten weiterbeschallt. Hierbei verdampft ein Grossteil des Aethers und teilweise das Aceton, wobei danach durch die Acetonunlöslichkeit des eingesetzten Lecithins die Abscheidung und Bildung der Phospholipidvesikel begünstigt wird. Die Restmengen an organischen Lösungmitteln werden im Rotationsverdampfer entfernt und die Liposomenlösung nach beschriebenem Verfahren filtriert. Das Präparat ist in Durchsicht wasserklar, zeigt im Turbidimeter jedoch einen ausgeprägten Tyndall-Effekt. Die Monodispersität der Lösung ist ausgezeichnet und die monolammellaren Vesikel zeigen einen mittleren Teilchendurchmesser von 40-50 nm. Liposomenlösungen dieser Art zeigten auch ohne Zusatz weiterer Stabilisatoren eine ausgezeichnete Stabilität.

Beispiel 6

200 g PL 100 werden in 300 ml Aether gelöst und unter Rühren zu einer Lösung von 3 g Diazepam in 300 ml Aceton gegeben (Ausfällungen können durch kurze Beschallung beseitigt werden). Hiernach wird mit 650 ml $N_2$-gesättigtem Wasser überschichtet und wie in Beispiel 1 ca. 45 Minuten beschallt. Eine eventuell auftretende Phasentrennung kann durch Hinzufügen von einigen ml Aceton rückgängig gemacht werden. Die verbliebenen organischen Lösungsmittelreste werden im Rotationsverdampfer entfernt und das Präparatvolumen wird nach Zugabe eines Phosphatpuffers (pH 7) mit Sorbitol (70 %ige wässrige Lösung) auf 1 l eingestellt. Die so erhaltene Liposomenlösung konnte direkt durch eine Membran von 0,22 μm Porengrösse filtriert und bei 120 °C/20 Minuten sterilisiert werden. Der mittlere Teilchendurchmesser betrug hiernach 60-70 nm und die elektronenmikroskopische Aufzeichnung zeigte eine ausgezeichnete Monodispersität.

Es wurden nach den oben beschriebenen Verfahren sowohl hydrophile wie auch lipophile Wirkstoffe in die organische bzw. wässrige Phase eingebracht und mit Erfolg in die Liposomensuspension eingeschlossen. Die Zugabe der Wirkstoffe in die entsprechende Phase erfolgt jeweils entweder direkt in die Lösung der Liposomenbildner oder in die beigefügte wässrige Phase vor der Beschallung.

**Patentansprüche**

1. Verfahren zur Herstellung von gegebenenfalls einen pharmakologischen Wirkstoff bzw. Stabilisatoren und Antioxidantien enthaltenden Liposomen lösungen, dadurch gekennzeichnet, daß man ein wässriges System, das einen Liposomenbildner und ein inertes, flüchtiges organisches Lösungsmittel oder ein Gemisch solcher Lösungsmittel enthält, mit Ultraschall behandelt, wobei man gegebenenfalls vor der Beschallung einen pharmakologischen Wirkstoff bzw. Stabilisatoren und Antioxidantien zusetzt.

2. Verfahren nach Anspruch 1, dadurch gekennzeichnet, daß der Liposomenbildner ein Lipid, insbesondere ein Phospholipid wie Lecithin ist.

3. Verfahren nach den Ansprüchen 1 oder 2, dadurch gekennzeichnet, daß das flüchtige organische Lösungsmittel ein niederer aliphatischer Alkohol, oder ein Aether ist.

**Claims**

1. A process for the manufacture of liposome solutions which optionally contain a pharmacologically active substance or stabilizers and antioxidants, characterized by treating an aqueous system, which contains a liposome former and an inert, volatile organic solvent or a mixture of such solvents, with ultrasound, whereby a pharmacologically active substance or stabilizers and antioxidants is/are optionally added prior to the acoustic irradiation.

2. A process according to claim 1, characterized in that the liposome former is a lipid, especially a phospholipid such as lecithin.

3. A process according to claims 1 or 2, characterized in that the volatile organic solvent is a lower aliphatic alcohol or an ether.

**Revendications**

1. Procédé de préparation de solutions de liposomes contenant le cas échéant un principe actif pharmacologique ou des stabilisateurs et des anti-oxydants, caractérisé en ce qu'un système aqueux qui contient un producteur de liposomes et un solvant organique volatil inerte ou un mélange de tels solvants est traité aux ultrasons, un principe actif pharmacologique ou des stabilisateurs et des anti-oxydants étant le cas échéant ajoutés avant la sonication.

2. Procédé selon la revendication 1, caractérisé en ce que le producteur de liposomes est un lipide, en particulier un phospholipide comme la lécithine.

3. Procédé selon les revendications 1 ou 2, caractérisé en ce que le solvant organique volatil est un alcool aliphatique inférieur ou un éther.